(19) Europäisches Patentamt / European Patent Office / Office européen des brevets

(11) **EP 3 896 071 B1**

(12) # EUROPEAN PATENT SPECIFICATION

(45) Date of publication and mention of the grant of the patent:
**01.11.2023 Bulletin 2023/44**

(21) Application number: **19892624.8**

(22) Date of filing: **09.12.2019**

(51) International Patent Classification (IPC):
***C07D 413/14*** *(2006.01)* ***A61K 31/4725*** *(2006.01)*
***A61P 25/18*** *(2006.01)*

(52) Cooperative Patent Classification (CPC):
**A61P 25/18; C07D 413/14**

(86) International application number:
**PCT/CN2019/123939**

(87) International publication number:
**WO 2020/114514 (11.06.2020 Gazette 2020/24)**

(54) **CRYSTALLINE FORM OF PROPIONAMIDE DERIVATIVE AND PREPARATION METHOD THEREFOR**

KRISTALLFORM EINES PROPANAMIDDERIVATS UND HERSTELLUNGSVERFAHREN DAFÜR

FORME CRISTALLINE D'UN DÉRIVÉ DE PROPANAMIDE ET PROCÉDÉ DE PRÉPARATION CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2018 CN 201811499965**

(43) Date of publication of application:
**20.10.2021 Bulletin 2021/42**

(73) Proprietor: **NHWA Pharma. Corporation**
**Jiangsu 221000 (CN)**

(72) Inventor: **HAO, Chao**
**Xuzhou, Jiangsu 221152 (CN)**

(74) Representative: **Müller, Christian Stefan Gerd**
**ZSP Patentanwälte PartG mbB**
**Hansastraße 32**
**80686 München (DE)**

(56) References cited:
**EP-A1- 3 901 149**

- **MINO R CAIRA ED - MONTCHAMP JEAN-LUC: "Crystalline Polymorphism of Organic Compounds", TOPICS IN CURRENT CHEMISTRY; [TOPICS IN CURRENT CHEMISTRY], SPRINGER, BERLIN, DE, vol. 198, 1 January 1998 (1998-01-01), pages 163-208, XP008166276, ISSN: 0340-1022, DOI: 10.1007/3-540-69178-2_5 [retrieved on 1999-02-26]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical Field

The present disclosure relates to a crystalline form of a propionamide derivative and a preparation method therefor.

### Background

Schizophrenia is the most serious and harmful disease of all psychiatric disorders. The latest research shows that the social burden of psychiatric disorders ranks first among diseases in China.

There are two main categories of existing schizophrenia drugs: typical anti-schizophrenia drugs and atypical anti-schizophrenia drugs. Although typical anti-schizophrenia drugs (such as Chlorpromazine and Haloperidol) have good effects on positive symptoms of schizophrenia, they have serious adverse reactions, such as extrapyramidal symptoms (EPS), tardive dyskinesia, and increased prolactin. Although atypical anti-schizophrenia drugs (such as Clozapine and Risperidone) significantly reduce the occurrence of extrapyramidal symptoms, adverse reactions such as extended QT interval and high prolactin still exist.

After decades of research, it has been found that five receptors such as $D_2$, 5-$HT_{1A}$, 5-$HT_{2A}$ and Hi play an important role in schizophrenia. WO2017084627A1 discloses a series of propionamide derivatives for treating schizophrenia, with the chemical name of 7-(3-(4-(6-fluorobenzo[d]isoazol-3-yl)piperidin-1-yl)propoxy)-2-methyl-3,4-dihydroisoquinolin-1(2H)-one , the structure is as follows:

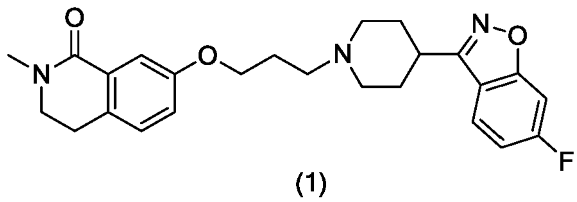

(1)

.

The crystal form structure of the pharmaceutical active ingredient often affects the chemical stability of the drug. Differences in crystalline form, preparation method and storage condition of the drug may lead to changes in the crystal form structure of the compound, sometimes accompanied by the production of crystal forms with other morphologies. Generally, amorphous drug products do not have regular crystal structures and often have other defects, such as poor product stability, difficult filtration, easy agglomeration, poor fluidity, etc. These differences often lead to difficulties in scale-up production. Therefore, it is necessary to improve various aspects of the properties of the compound through the morphology of the crystal form, and to perform intensive study to find a new crystal form with higher crystal form purity and good chemical stability.

### Summary

The technical problem to be solved herein is to provide a crystalline form of a propionamide derivative and a preparation method therefor, i.e., the Crystal Form A of the compound of formula (1), which has good crystalline form stability and chemical stability, and can be better applied in clinic.

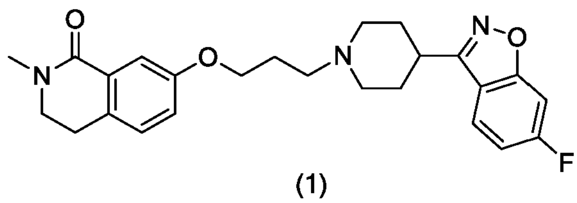

(1)

In an aspect, provided is Crystal Form A of a compound of formula (1), characterized in that, the Crystal Form A has characteristic peaks at 4.46, 11.30, 13.59, 18.17, 21.38, 22.03, 25.89 in the X-ray powder diffraction pattern obtained by Cu-K$\alpha$ radiation and represented by diffraction angle 2$\theta$ angle, wherein the error range may be $\pm$0.3, $\pm$0.2 or $\pm$0.1.

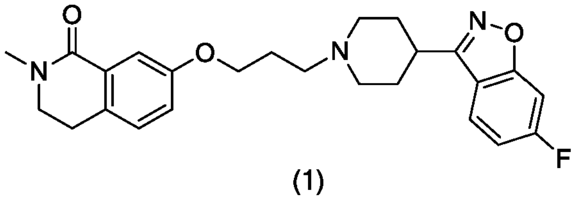

(1)

In an embodiment of the present invention, the Crystal Form A has characteristic peaks, represented by 2θ angle, at 4.46, 9.01, 11.30, 12.55, 13.59, 14.21, 15.67, 16.45, 17.25, 18.17, 18.54, 18.85, 19.51, 20.89, 21.38, 22.03, 22.93, 24.43, 25.07, 25.89, 27.09, 27.81, 28.14, 29.31, 30.02 and 31.85, wherein the error range may be ±0.3, ±0.2 or ±0.1.

In an embodiment of the present invention, the Crystal Form A has a Raman spectrum with characteristic peaks at 3065.5±2 $cm^{-1}$, 2958.4±2 $cm^{-1}$, 1607.8±2 $cm^{-1}$, 1447.8±2 $cm^{-1}$, 1320.2±2 $cm^{-1}$, 1271.5±2 $cm^{-1}$, 1125.3±2 $cm^{-1}$, 1009.3±2 $cm^{-1}$, 918.94±2 $cm^{-1}$, 714.8±2 $cm^{-1}$, 309.2±2 $cm^{-1}$, 233.2±2 $cm^{-1}$.

In an embodiment of the present invention, the Crystal Form A has a DSC with melting endothermic peak values selected from the group consisting of 116.4 to 122.0°C, preferably 119.4°C.

In an embodiment of the present invention, the Crystal Form A accords with one or more of the following solid state characteristics:

(I) a powder X-ray diffraction pattern substantially according with Figure 1;
(II) a DSC thermogram substantially according with Figure 2;
(III) a Raman spectrum pattern substantially according with Figure 3.

In another aspect, provided is a method for preparing the Crystal Form A, which is selected from the group consisting of Method 1 and Method 2:

**Method 1**

① dissolving a compound of formula (1) in a solvent to give a solution containing the compound of formula (1),
② removing the solvent in the solution obtained in step ① by evaporation to give a precipitate;

**Method 2**

① identical to step ① of Method 1;
② obtaining a precipitate from the solution obtained in step ① by precipitation method.

In an embodiment according to Method 1, the solvent in step ① is selected from the group consisting of one or more of $C_{1-6}$ alcohol, ester, ketone, ether, halogenated hydrocarbon, nitrile, $C_{5-10}$ saturated hydrocarbon, water, N-methyl-2-pyrrolidone, N, N-dimethylformamide and dimethylsulfoxide.

Specifically, in an embodiment according to Method 1, the $C_{1-6}$ alcohol is selected from the group consisting of one or more of methanol, ethanol, n-propanol, isopropyl alcohol or n-butanol; the ester is selected from the group consisting of one or more of ethyl acetate, n-propyl acetate, isopropyl acetate or isobutyl acetate; the ketone is selected from the group consisting of one or more of acetone, 2-butanone, pentan-2-one, pentan-3-one, hexan-2-one or hexan-3-one; the ether is selected from the group consisting of one or more of methyl tert-butyl ether, ethyl ether, tetrahydrofuran, diisopropyl ether or 1,4-dioxane; the nitrile is selected from the group consisting of acetonitrile; the halogenated hydrocarbon is selected from the group consisting of one or more of dichloromethane or chloroform; the $C_{5-10}$ saturated hydrocarbon is selected from the group consisting of one or more of n-pentane, n-hexane, cyclohexane or n-heptane.

In an embodiment according to Method 1, the solvent in step ① is selected from the group consisting of one or more of chloroform, methanol, ethanol, ethyl acetate, acetone or n-heptane.

In an embodiment according to Method 1, the solvent in step ① is a mixed solvent of $C_{5-10}$ saturated hydrocarbon and any one or more selected from the group consisting of alcohol, ketone and halogenated hydrocarbon, a mixed solvent of water and any one or more selected from the group consisting of ketone, alcohol, a mixed solvent of alcohol and ester.

In an embodiment according to Method 1, the mixed solvent is selected from the group consisting of ethanol/ethyl acetate, n-heptane/ethanol, n-heptane/acetone, n-heptane/chloroform, n-hexane/ethanol, n-hexane/ acetone, n-hexane/ chloroform.

In an embodiment according to Method 1, the ethanol has a water content of ≤5%(v/v).

In an embodiment according to Method 1, the step ① further comprises a heating process. The heating temperature of the heating process is selected from the group consisting of the temperature lower than the boiling point of the solvent used in step ①.

In an embodiment according to Method 1, the step ① further comprises a heating process. The heating temperature of the heating process is selected from the group consisting of 20 to 80°C, preferably 20 to 60°C.

In an embodiment according to Method 1, the evaporation method of solvent in step ② is preferably vacuum evaporation method. The vacuum evaporation method is preferably performed with rotatory evaporator method.

In an embodiment according to Method 1, the evaporation method is evaporating the solvent of step ② in airflow. The airflow is preferably flow of air or inert gas. The inert gas is preferably argon or nitrogen flow.

In an embodiment according to Method 2, the precipitation method in step ② is selected from the group consisting of cooling method or precipitant method.

In an embodiment according to Method 2, the cooling method in step ② is subjecting the solution obtained in step ① to cooling process to precipitate the crystals out.

In an embodiment according to Method 2, the cooling process is lowering the temperature of the solution obtained in step ① to -10 to 15°C, preferably -10 to 9°C, more preferably 0 to 9°C.

In another embodiment according to Method 2, the cooling process is lowering the temperature of the solution obtained in step ① to 0 to 60°C, preferably 10 to 40°C, more preferably 15 to 25°C.

In another embodiment according to Method 2, the cooling process is lowering the temperature to a temperature which is 20 to 100°C lower than that of the solution obtained in step ①, preferably 30 to 100°C lower than that of the solution obtained in step ①, more preferably 60 to 100°C lower than that of the solution obtained in step ①.

In an embodiment according to Method 2, the precipitant method in step ② is adding a precipitant of the compound of formula (1) into the solution obtained in step ① to precipitate the crystals out. The precipitant is selected from the group consisting of $C_{5-10}$ saturated alkane or water.

In the above embodiments according to Method 2, the $C_{5-10}$ saturated alkane is selected from the group consisting of one or more of n-pentane, n-hexane, n-heptane.

In an embodiment according to Method 2, the step ② further comprises a precipitation time for obtaining precipitates from the solution obtained in step ①. The precipitation time is selected from the group consisting of 0-120 min, 0-60 min, 0-30 min, 0-10 min, 0-5 min, 0-2 min, 0-30 sec or 0 sec, preferably 0-10 min, 0-5 min, 0-2 min, 0-30 sec or 0 sec, wherein the "0" or "0 sec" refers to the time point when the precipitant is immediately added.

In an embodiment according to Method 2, the precipitation time in step ② is that for maximum precipitating amount. The precipitation time for maximum precipitating amount is selected from the group consisting of 0-90 min, 0-80 min, 0-70 min or 0-60 min, preferably 0-70 min or 0-60 min, and most preferably 0-60 min. The "0" refers to the time point when the precipitant is completely added. The maximum precipitating amount means that the compound of formula (1) is precipitated out completely as precipitates from the solution obtained in step ① or at least 85% of the amount of the compound of formula (1) (mass ratio of precipitating amount to dissolved amount of compound of formula (1)) is precipitated out from the solution obtained in step ①.

In a preferable embodiment according to the present invention, Method 1 or Method 2 further comprises the following steps:

③ separating the precipitates obtained in step ② of Method 1 or Method 2;
④ drying the solid obtained in step ③.

In an embodiment, step ③ further comprises a separating temperature. The separating temperature is selected from the group consisting of 0 to 60°C, preferably 5 to 40°C, more preferably 15 to 25°C.

In an embodiment, step ④ further comprises a drying temperature. The drying temperature is selected from the group consisting of 0 to 60°C, preferably 5 to 40°C, more preferably 15 to 25°C.

Disclosed is also a Crystal Form B of a compound of formula (1), characterized in that, the Crystal Form B has an X-ray powder diffraction pattern obtained by Cu-Kα radiation and represented by diffraction angle 2θ angle, having characteristic peaks at 8.46, 10.35, 10.99, 13.50, 18.13, 24.13, 27.82, 29.23, wherein the error range may be ±0.3, ±0.2 or ±0.1.

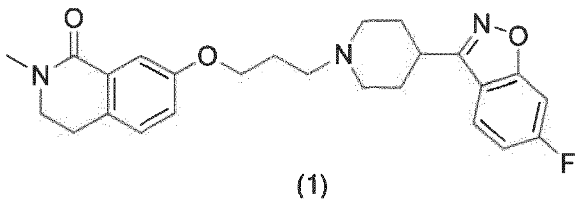

(1)

In an embodiment, the Crystal Form B has a Raman spectrum with characteristic peaks at 3082.3±2 $cm^{-1}$, 2927.6±2 $cm^{-1}$, 1610.1±2 $cm^{-1}$, 1515.8±2 $cm^{-1}$, 1446.4±2 $cm^{-1}$, 1352.6±2 $cm^{-1}$, 1261.2±2 $cm^{-1}$, 1171.5±2 $cm^{-1}$, 914.4± 2$cm^{-1}$, 709.7±2 $cm^{-1}$, 307.0±2 $cm^{-1}$, 257.6±2 $cm^{-1}$.

In an embodiment, the Crystal Form B has a DSC with 2 melting endothermic peak values and 1 exothermic peak, wherein the 1st endothermic peak value is 101.6°C; the 2nd endothermic peak value is in a range of 116.2 to 120.6°C, preferably 119.0°C; and the exothermic peak value is 104.7°C.

In an embodiment, the Crystal Form B accords with one or more of the following solid state characteristics:

(I) a powder X-ray diffraction pattern substantially according with Figure 4;
(II) a DSC thermogram substantially according with Figure 5;
(III) a Raman spectrum pattern substantially according with Figure 6.

Disclosed is also a method for preparing the Crystal Form B, which is selected from the group consisting of self-melting recrystallization method and specifically comprises the following steps:

① melting the compound of formula (1) completely under the condition of elevated temperature;
② recrystallizing the sample after melting in step ① under the condition of lowered temperature.

In an embodiment, the condition of elevated temperature in step ① is selected from the group consisting of 120 to 140°C, preferably 120°C, 125°C, 130°C, 135°C or 140°C, more preferably 125°C.

In an embodiment, the condition of lowered temperature in step ② is selected from the group consisting of 20 to 70°C, preferably 45 to 60°C, more preferably 60°C.

In a yet further aspect, provided is a pharmaceutical composition, comprising the compound of formula (1) in the form of Crystal Form A as active ingredient, and a pharmaceutically acceptable excipient, carrier, adjuvant, solvent or a combination thereof.

In an embodiment, the active ingredient comprises at least 50-99% Crystal Form A, preferably at least 70-99% Crystal Form A, more preferably at least 90-99% Crystal Form A.

In an embodiment, the Crystal Form A is present in the active ingredient in a substantially pure form.

In an embodiment, the pharmaceutical composition can be administered by any suitable route, for example by oral administration in the form of capsule, by parenteral administration in the form of injection liquid, by topical administration in the form of paste or lotion, by rectal administration in the form of suppository, by transdermal administration in the form of patch delivery system. In a preferable embodiment, the pharmaceutical composition is administered orally.

Provided is also use of the Crystal Form A according to the present invention for the manufacture of a pharmaceutical composition. Preferably, the pharmaceutical composition is useful for treating and/or preventing a psychiatric disorder. The psychiatric disorder is preferably schizophrenia.

Provided is also the Crystal Form A according to the present invention for use in treating a disease, particularly in treating and/or preventing a psychiatric disorder. The psychiatric disorder is preferably schizophrenia.

## Detailed Description

In the present specification and claims, unless otherwise stated, the scientific and technical terms used herein have the meanings commonly understood by those skilled in the art. However, for better understanding of the present invention, the following definitions and interpretations of relevant terms are provided. In addition, when the definitions and interpretations of the terms provided herein are inconsistent with those commonly understood by those skilled in the art, the definitions and interpretations of the terms provided herein shall prevail.

The term "alcohol" as used herein refers to a group derived from "$C_{1-6}$ alkyl", of which one or more hydrogen atoms are substituted by one or more "hydroxyl groups", and the "$C_{1-6}$ alkyl" is as defined above. Specific examples include but are not limited to methanol, ethanol, n-propanol or isopropyl alcohol.

The term "ester" as used herein refers to a compound with carbon atom number of 15 or less which is formed by the reaction of organic acid and alcohol or phenol to be dehydrated, or a lower ester compound with the functional

group of -C(O)O- and carbon atom number of 15 or less. Specific examples include but are not limited to methyl acetate, ethyl acetate, dimethyl phthalate, butyl acetate or propyl acetate.

The term "ether" as used herein refers to a chain-like or cyclic compound containing an ether bond -O- and carbon atom number of 1-10. Specific examples include but are not limited to ethyl ether, diisopropyl ether, propanediol methyl ether, tetrahydrofuran, methyl tert-butyl ether or 1,4-dioxane.

The term "halogenated hydrocarbon" as used herein refers to a group derived from "$C_{1-6}$ alkyl", of which one or more hydrogen atoms are substituted by one or more "halogen atoms", and the "$C_{1-6}$ alkyl" is as defined above. Specific examples include but are not limited to methyl chloride, dichloromethane, dichloroethane, chloroform or carbon tetrachloride.

The term "ketone" as used herein refers to a compound in which a carbonyl group (-C(O)-) is connected to two hydrocarbon groups. According to different hydrocarbon groups in the molecule, ketones can be divided into aliphatic ketone, alicyclic ketone, aromatic ketone, saturated ketone and unsaturated ketone. Specific examples include but are not limited to acetone, acetophenone, methyl isobutyl ketone or methyl pyrrolidone.

The term "nitrile" as used herein refers to a group derived from a "$C_{1-6}$ alkyl" of which one or more hydrogen atoms are substituted by one or more "cyano groups", and the "cyano group" and "$C_{1-6}$ alkyl" are as defined above. Specific examples include but are not limited to acetonitrile or propionitrile.

The term "saturated hydrocarbon" used herein refers to $C_{5-10}$ chain-like or cyclic alkane, the carbon atoms in the molecule are all connected by single bond, and the rest of the valence bonds are all bound with hydrogens. Specific examples include but are not limited to n-pentane, n-hexane, cyclohexane and n-heptane.

The term "mixed solvent" as used herein refers to a solvent obtained by mixing one or more different types of solvents in a certain ratio, and the certain ratio is 0.05:1-1:0.05, preferably 1:1, 1:2, 1:3, 1:4, 1:5, 1:8, 1:10.

The term "precipitant" as used herein refers to "antisolvent" or "anti-solvent", which means when a certain component is separated or removed, the substance is dissolved in a suitable solvent in advance, and a solvent which is insoluble with the component to be separated is added. The precipitant is miscible with the solvent in which the compound of formula (1) is dissolved.

The term "boiling point" as used herein refers to the boiling point or azeotropic point of a pure solvent or a mixed solvent.

The term "X-ray powder diffraction pattern" or "XRPD" as used herein refers to that according to the Bragg equation $2d \sin \theta = n\lambda$ (where $\lambda$ is the wavelength of X ray, $\lambda$=1.54056Å, and the diffraction order n is any positive integer, generally the first-order diffraction peak, n=1), when X ray is incident on the atom surface of a crystal or part of the crystal sample with d lattice plane spacing at swept angle $\theta$ (complementary angle of the incident angle, also known as Bragg angle), the Bragg equation can be satisfied, and this set of X-ray powder diffraction pattern can be determined.

The term "2$\theta$" or "2$\theta$ angle" as used herein refers to diffraction angle, where $\theta$ is Bragg angle, the unit is ° or degree, and the error range of 2$\theta$ is $\pm$0.1 to $\pm$0.5, preferably $\pm$0.1 to $\pm$0.3, more preferably $\pm$0.2.

The term "interplanar spacing" or "interplanar spacing (d value)" as used herein refers to that the spatial lattice selects three non-parallel unit vectors a, b, and c which are connected with two adjacent lattice points, and the matrix is divided into juxtaposed parallelepiped units by the unit vectors, which are known as interplanar spacing. The spatial lattice is divided according to the lines of the determined parallelepiped units to obtain a set of linear grids, which are known as spatial lattice or lattice. The spatial lattice and lattice use geometric points and lines to reflect the periodicity of the crystal structure, respectively. Different crystal planes have different interplanar spacing (that is, the distance between two adjacent parallel crystal planes); the unit is Å or Angstrom.

The term "differential scan calorimetry" or "DSC" as used in herein measures the transition temperature when a crystal absorbs or releases heat due to a change in the crystal structure or crystal melting. For the same crystal form of the same compound, in continuous analysis, the thermal transition temperature and melting point error can be within about 5°C, usually within about 3°C. When describing a compound with a given DSC peak or melting point, it refers to the DSC peak or melting point $\pm$5°C. The term "substantially" also takes this temperature change into account. DSC provides an auxiliary method to distinguish different crystal forms. Different crystal morphologies can be identified according to their different transition temperature characteristics. It should be noted that, for a mixture, its DSC peak or melting point may vary in a larger range. In addition, due to the decomposition within the melting process, the melting temperature is relevant with the heating rate.

The term "Fourier Raman spectrum (FT-Raman)" as used herein is generally used to investigate the structure and chemical bonds of molecules and can also be used as a method to characterize and identify chemical species. Fourier Raman spectrum used herein for characterizing the molecular structure and crystal form of FT-Raman may have the peak position error range of $\pm$2 $cm^{-1}$.

Compared with the prior art, the technical solution according to the present invention has the following advantages: Studies have shown that the Crystal Form A of the compound of formula (1) according to the present invention has high purity and good crystalline stability; HPLC purity changes are small, and chemical stability is high. The Crystal Form A of the compound of formula (1) obtained according to the present invention can meet the medicinal requirements

of production, transportation and storage, the production process is stable, repeatable and controllable, and can be adapted to industrial production.

## Brief Description of the Drawings

Figure 1 is the X-ray powder diffraction pattern of the Crystal Form A of the compound of formula (1).
Figure 2 is the DSC spectrum of the Crystal Form A of the compound of formula (1).
Figure 3 is the Raman spectrum of the Crystal Form A of the compound of formula (1).
Figure 4 is the X-ray powder diffraction pattern of the Crystal Form B of the compound of formula (1).
Figure 5 is the DSC spectrum of the Crystal Form B of the compound of formula (1).
Figure 6 is the Raman spectrum of the Crystal Form B of the compound of formula (1).

## Examples

The present invention will be explained in more details below by reference to the Examples, and the present Examples are only used to illustrate the technical solutions of the present invention rather than any limitation to the scope.

Instruments used in the experiments and test conditions:

1. X-ray Powder Diffraction Spectrum (XRPD)

Instrument model: Bruker D8 Focus Powder X-ray Diffractometer.
X-ray parameter: Cu/Kα (λ=1.540598Å)
Voltage: 40 kilovolts (kV)
Electricity: 40 milliamperes (mA)
Scan range: from 3.0 to 60 degrees
Scan step: 0.02 degrees
Scan step rate: 0.5 seconds/step

2. Differential Scanning Calorimeter (DSC)

Instrument model: NETZSCH DSC 200F3 differential scanning calorimeter
Purge gas: nitrogen
Heating rate: 10.0 K/min
Temperature range: 30-250°C

3. FT-Raman Spectrometer (FT-RM)

Instrument model: Thermo Scientific DXR Smart Raman spectrograph
Diaphragm: 50 μm
Exposure time: 10 s
Exposure number: 32 times
Laser: 780 nm
Laser energy: 150 mw

4. High Performance Liquid Chromatograph (HPLC)
Instrument model: Agilent 1260 (DAD) binary pump liquid chromatography
Chromatographic column: Agilent Eclipse XDB (4.6*150mm,5μm) C18 column
Mobile phase:

A: 0.01 mol/L $KH_2PO_4$ (pH 3.0)-methanol (90:10)

(continued)

| | | | |
|---|---|---|---|
| B: | methanol-water (90:10) | | |
| Flow rate: | 1.0 ml/min | Column temperature: | 35°C |
| Wavelength: | 210 nm | Injection volume: | 5 μl |

Gradient conditions (volume ratio):

| Time (min) | A (%) | B (%) |
|---|---|---|
| 0 | 80 | 20 |
| 60 | 20 | 80 |
| 61 | 80 | 20 |

Reagents used in the experiments:
Methanol (analytically pure), acetone (analytically pure), ethanol (analytically pure), n-hexane (analytically pure) are all purchased from Shanghai Lingfeng Chemical Reagent.

**Example 1. Preparation of the compound of formula (1)**

The compound of formula (1) can be prepared according to the method of PCT/CN2016/106591 (see the method documented in Example 1 and Example 5).

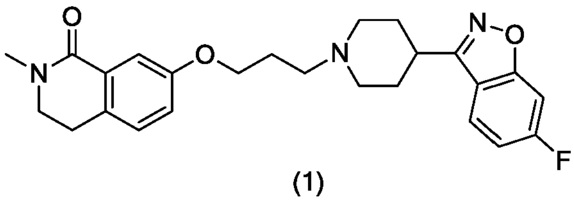

**(1)**

**Example 2. Preparation of Crystal Form A**

1 g of the compound of formula (1) was weighed and added into an eggplant bottle, to which was then added 60 ml of methanol. The solvent was rotated off under the reduced pressure in the condition of 0.09 MPa vacuum degree and 40°C, and the solid was collected, which was then dried over night at 60°C to give the final product. LC purity was 97.2%. The X-ray Powder Diffraction is shown in Figure 1, the DSC spectrum is shown in Figure 2, and the Raman spectrum is shown in Figure 3. During the DSC heating, the initial point of endothermic peak is 116.4°C and end point is 122.0°C, with the peak value of 119.4°C. The crystal form is defined as Crystal Form A with characteristic peak positions as shown in Table 1 below.

Table 1

| 2θ angle/degree | d value /Å | Intensity (%) |
|---|---|---|
| 4.46 | 19.80 | 11.8 |
| 9.01 | 9.81 | 6.2 |
| 11.30 | 7.83 | 17.6 |
| 12.55 | 7.05 | 6.3 |
| 13.59 | 6.51 | 100.0 |
| 14.21 | 6.23 | 17.6 |
| 15.67 | 5.65 | 6.2 |
| 16.45 | 5.38 | 13.4 |
| 17.25 | 5.14 | 34.5 |

(continued)

| 2θ angle/degree | d value /Å | Intensity (%) |
|---|---|---|
| 18.17 | 4.88 | 84.6 |
| 18.54 | 4.78 | 11.2 |
| 18.85 | 4.70 | 7.9 |
| 19.51 | 4.55 | 9.7 |
| 20.89 | 4.25 | 45.3 |
| 21.38 | 4.15 | 87.8 |
| 22.03 | 4.03 | 100.0 |
| 22.93 | 3.88 | 24.8 |
| 24.43 | 3.64 | 8.9 |
| 25.07 | 3.55 | 15.3 |
| 25.89 | 3.44 | 63.8 |
| 27.09 | 3.29 | 14.7 |
| 27.81 | 3.21 | 11.4 |
| 28.14 | 3.17 | 9.7 |
| 29.31 | 3.05 | 7.1 |
| 30.02 | 2.97 | 12.3 |
| 31.85 | 2.81 | 15.5 |

**Example 3. Preparation of Crystal Form A**

1 g of the compound of formula (1) was weighed and added into an eggplant bottle, to which was then added 35 ml of acetone. The solvent was rotated off under the reduced pressure in the condition of 0.09 MPa vacuum degree and 35°C, and the solid was collected, which was then dried over night at 60°C to give the final product. The product was determined by X-ray powder diffraction as the Crystal Form A.

**Example 4. Preparation of Crystal Form A**

1 g of the compound of formula (1) was weighed and added into an eggplant bottle, to which was then added 80 ml of ethanol and 20 ml of n-hexane. The solvent was rotated off under the reduced pressure in the condition of 0.09 MPa vacuum degree and 40°C, and then the solid was collected, which was then dried over night at 60°C to give the final product. The product was determined by X-ray powder diffraction as the Crystal Form A.

**Example 5. Preparation of Crystal Form A**

0.5 g of the compound of formula (1) was weighed and added into 10 ml of acetone, which was heated to reflux. When the solution was clear, heating was continued with stirring for reflux. Stirring was stopped after 30 min and the reaction mixture was allowed to stand for spontaneous cooling to room temperature and large amount of solid precipitated. Filtration was performed and the collected filter cake was dried overnight in a vacuum oven at 60°C to give the final product. The product was determined by X-ray powder diffraction as the Crystal Form A.

**Example 6. Preparation of Crystal Form A**

0.5 g of the compound of formula (1) was weighed and added into 12 ml of acetone and 5 ml of water, which was heated to reflux. When the solution was clear, heating was continued with stirring for reflux. Stirring was stopped after 30 min and the reaction mixture was allowed to stand for spontaneous cooling to room temperature and large amount of solid precipitated. Filtration was performed and the collected filter cake was dried overnight in a vacuum oven at 60°C to give the final product. The product was determined by X-ray powder diffraction as the Crystal Form A.

**Example 7. Preparation of Crystal Form A**

0.5 g of the compound of formula (1) was weighed and added into 5.0 ml of anhydrous ethanol to give a clear solution. 100 ml of water was quickly poured into the clear solution and large amount of white solid precipitated. The suspension was allowed to stand for settling. Filtration was performed and the filter cake was collected and dried overnight in a vacuum oven at 60°C to give the final product. The product was determined by X-ray powder diffraction as the Crystal Form A.

**Example 8. Preparation of Crystal Form A**

0.5 g of the compound of formula (1) was weighed and added into 5.0 ml of anhydrous ethanol to give a clear solution. 100 ml of n-hexane was quickly poured into the clear solution and large amount of white solid precipitated. The suspension was allowed to stand for settling. Filtration was performed and the filter cake was collected and dried overnight in a vacuum oven at 60°C to give the final product. The product was determined by X-ray powder diffraction as the Crystal Form A.

**Example 9. Preparation of Crystal Form B** (not according to the invention)

About 500 mg of the compound of formula (1) was weighed in a small beaker, which was placed in a vacuum oven at 120°C. After the sample melt completely, the small beaker was quickly transferred to 60°C condition for quick cooling and maintained at 60°C condition for 8 h until complete crystallization to give the final product. LC purity was 96.7%. The X-ray Powder Diffraction is shown in Figure 4, the DSC spectrum is shown in Figure 5, and the Raman spectrum is shown in Figure 6. During the DSC heating process, there were two endothermic peaks and 1 exothermic peak, wherein the 1$^{st}$ endothermic peak value was 101.6°C; the 2$^{nd}$ endothermic peak had an initial point of 116.2°C and an end point of 120.6°Cand the peak value of 119.0°C; and the exothermic peak value was 104.7°C. The product was determined by X-ray powder diffraction as the Crystal Form B with characteristic peak positions as shown in Table 2 below.

Table 2

| 2θ angle/degree | d value/Å | Intensity (%) |
|---|---|---|
| 8.46 | 9.82 | 3.9 |
| 10.35 | 8.54 | 30.5 |
| 10.99 | 8.05 | 23.4 |
| 13.01 | 6.80 | 11.1 |
| 13.50 | 6.55 | 23.1 |
| 14.49 | 6.11 | 8.1 |
| 16.49 | 5.37 | 10.3 |
| 17.06 | 5.19 | 6.9 |
| 17.56 | 5.05 | 10.2 |
| 18.13 | 4.89 | 99.5 |
| 20.31 | 4.37 | 5.8 |
| 20.82 | 4.26 | 13.8 |
| 21.11 | 4.21 | 14.7 |
| 21.86 | 4.06 | 8.9 |
| 22.35 | 3.97 | 9.6 |
| 22.99 | 3.87 | 7.1 |
| 24.13 | 3.69 | 100.0 |
| 25.05 | 3.55 | 7.6 |
| 26.66 | 3.34 | 5.2 |

(continued)

| 2θ angle/degree | d value/Å | Intensity (%) |
|---|---|---|
| 27.66 | 3.22 | 7.7 |
| 27.82 | 3.20 | 8.8 |
| 29.23 | 3.05 | 8.2 |

**Example 10. Preparation of Crystal Form B** (not according to the invention)

About 500 mg of the compound of formula (1) was weighed in a small beaker, which was placed in a vacuum oven at 120°C. After the sample melt completely, the small beaker was quickly transferred to room temperature (about 25°C) condition and maintained at room temperature condition for 48 h until complete crystallization to give the final product. The product was determined by X-ray powder diffraction as the Crystal Form B.

**Example 11. Crystalline Stability**

Experimental methods:

By referring to the Guidelines for the Stability test of raw drugs and preparations of the Chinese Pharmacopoeia 2015 Edition of the Four General Rule 9001 (see page 354 of the Chinese Pharmacopoeia Part 4), Specifically, Crystal Form A and Crystal Form B were respectively tested for stability factors at high humidity (R.H. 92.5%), high temperature (60°C) and light (4500±500 lx) conditions. The samples were taken at day 5 and day 10 respectively for PXRD (polycrystalline X ray diffraction) detection and HPLC content (w/w, %) determination, and the results were compared with that of day 0.

Table 3 Crystal Form A and Crystal Form B stability factors tests

| Testing condition | Crystal Form A | | Crystal Form B | |
|---|---|---|---|---|
| | Crystal form | Content (%) | Crystal form | Content (%) |
| Day 0 | Crystal Form A | 97.21 | Crystal Form B | 96.70 |
| Day 1 0 at high temperature | Crystal Form A | 97.15 | Crystal Form B + Crystal Form A | 96.65 |
| Day 10 at light | Crystal Form A | 97.13 | Crystal Form B + Crystal Form A | 96.69 |
| Day 1 0 at high humidity | Crystal Form A | 97.18 | Crystal Form B + Crystal Form A | 96.67 |

Experimental Results:

According to the data in Table 3, with respect to Crystal Form A, not only the morphology of crystal form was stable under the conditions of high humidity for 10 days, high temperature for 10 days and light for 10 days, but also the chemical properties were stable, where, as compared to day 0, there was almost no change in content, and all the content could reach 97.0% or more.

With respect to Crystal Form B, the morphology of crystal could not remain stable under any experimental conditions of high humidity for 10 days, high temperature for 10 days and light for 10 days, and Crystal Form B gradually changed to Crystal Form A, but the chemical properties were stable, and the content did not change as compared to day 0. All the content could reach 96.0% or more.

In summary, it can be seen that the stability of Crystal Form A is better than that of Crystal Form B.

**Example 12. Investigation of the mechanical stress of the crystal form**

Experimental methods:

About 1000 mg of Crystal Form A sample and Crystal Form B sample were weighed in the agate mortar of the ball mill, the speed of the ball mill was set to be 400 r/min and to stop for 15 min every 30 min of grinding. Samples were taken when the ball milled at 30 min, 4 h and 6 h respectively, and then for PXRD test. The change of crystal form was observed, and the experiment was carried out parallelly in duplicate. The specific test results were shown in Table 4.

Table 4 Crystal Form A and Crystal Form B mechanical stress test

| Mortar | Sample size (mg) | 30 min | 4h | 6h |
|---|---|---|---|---|
| 1 | 1000.91 | Crystal Form A | Crystal Form A | Crystal Form A |
| 2 | 1000.74 | Crystal Form A | Crystal Form A | Crystal Form A |
| 3 | 1000.83 | Crystal Form B | Crystal Form B+ Crystal Form A | Crystal Form B+ Crystal Form A |
| 4 | 1000.56 | Crystal Form B | Crystal Form B+ Crystal Form A | Crystal Form B+ Crystal Form A |

It can be seen from Table 4 that Crystal Form A, after the test of being ball milled for 30 min, 4 h and 6 h respectively, remained stable under ball milling pressure conditions, and its crystal form did not change. Crystal Form B, after the test of being ball milled for 30 min, 4 h and 6 h respectively, gradually transformed into crystal A under ball milling pressure conditions, and its crystal form changed significantly, indicating that Crystal Form A is more suitable for the pulverization process of pharmaceutical industrialization than Crystal Form B, and is suitable for large-scale pharmaceutical industry production.

**Example 13. Pharmacokinetic investigation of Crystal Form A and Crystal Form B**

This Example provides a comparative study regarding the pharmacokinetics of Crystal Form A and Crystal Form B according to the present invention in beagle dogs.

Testing sample

Freshly prepared Crystal Form A, Crystal Form B (Crystal Form A was prepared by referring to the method disclosed in Example 1, and Crystal Form B was prepared by referring to the method disclosed in Example 9, wherein the LC purity of Crystal Form A was 97.0%, and the LC purity of Crystal Form B was 96.9%).

Test animals

There were 12 beagle dogs for the experiment, half male and half female, weighing 11.0-14.1 kg, provided by the Teaching and Experimental Ground of Agricultural College of Shanghai Jiao Tong University. The animals were raised in single cage, and the feeding amount can be adjusted according to the weight or feed intake of the animals. The animals can drink water freely, with 12/12 h light/dark cycle adjustment, constant temperature of 23±1°C, humidity of 50-60%. On the day of administration, the experimental animals were fasted overnight before administration.

Test equipment and materials

Waters 2690 High performance liquid chromatograph, MicroMass ZMD 400 Electrospray mass spectrometer (ESI), Beckman High-speed refrigerated centrifuge, Eppendorf centrifuge.

Preparation of Testing sample capsule

The freshly prepared Crystal Form A and Crystal Form B were filled into capsule shells (commercially available) and stored in a dry place at room temperature for experimental use.

Test methods

Grouping

Beagle dogs were randomly divided into groups according to their body weight and were divided into Crystal Form A group (n=6, half male and half female) and Crystal Form B group (n=6, half male and half female).

Administration

The animals were weighed on the day of administration, and the dosage was determined according to their body weight. The above-mentioned grouped beagle dogs were administered according to the method in Table 5 below.

Table 5

| Group/Stage | Testing sample | Pre-treatment | Administration route | dosage (mg/kg) |
|---|---|---|---|---|
| Crystal Form A Group | Crystal Form A | Food intake | PO | 5 |
| Crystal Form B Group | Crystal Form B | Food intake | PO | 5 |

Sample collection and processing

1 mL of whole blood was collected from the cephalic vein at blood collection time points of 0.0830, 0.250, 0.500, 1.00, 2.00, 4.00, 8.00, 24.00 h after administration. After the blood samples were collected, they were immediately transferred to labeled, heparin sodium-containing (20 μL, 1000 IU) anticoagulant centrifuge tubes, which were inverted several times and then centrifuged (1,500 g, 10 min, 4°C) to collect plasma.

Sample analysis

The analytical method was performed by liquid chromatography tandem triple quadrupole mass spectrometry (LC MS/MS). The lower limit of quantification (LLOQ) of the compound of formula (1) in dog plasma was 2.0 ng/mL, and the upper limit of quantification (ULOQ) was 1000 ng/ mL.

Data analysis

WinNonlin™ Version 6.2.1 (Pharsight, Mountain View, CA) pharmacokinetic software was used to process the plasma drug concentration data of Crystal Form A and Crystal Form B in an extravascular model (extravascular). The peak concentration ($C_{max}$) and the peak time ($T_{max}$) were obtained from the plasma concentration-time curve graph. The log-linear trapezoidal method (see: Gabrielsson J, Weiner D. Pharmacokinetic and pharmacodynamic data analysis: concepts and applications[M]. CRC Press, 2001, pages 141-146) was used to calculate the following parameters: elimination phase half-life ($T_{1/2}$), mean retention time extrapolated from zero time point to infinity ($MRT_{0-inf}$), mean retention time from zero time point to the last detectable concentration time point ($MRT_{0-last}$), the area under the plasma concentration-time curve from the zero time point to the last detectable concentration time point ($AUC_{0-last}$), and the area under the plasma concentration-time curve extrapolated from the zero time point to infinity($AUC_{0-inf}$).

In this experiment, the error between the actual blood collection time at all blood collection time points and the blood collection time specified in the experimental protocol was within the specified range, and thus the theoretical blood collection time was used to calculate the pharmacokinetic parameters.

The experimental data was expressed as the mean (Mean) ± standard deviation (S.D.). The excel software t-test was used for statistical comparison. The relevant data between the different crystal form administration groups were analyzed and compared to determine whether there was significant statistical difference. Wherein "*" was P<0.05, which meantthat Crystal Form A had significantly differences respectively compared to Crystal Form B. The specific test results were shown in Table 6.

Table 6 Comparison of pharmacokinetic parameters of Crystal Form A and Crystal Form B

| Crystal form type / pharmacokinetic parameters | Crystal Form **A** ( $\bar{x}$±s, n=6) | Crystal Form **B** ( $\bar{x}$±s, n=6) |
|---|---|---|
| $C_{max}$ (ng/mL) | 645±37.23* | 426±32.75 |
| $T_{max}$ (hr) | 2.6* | 1.7 |
| $T_{1/2}$ (hr) | 2.9 | 2.1 |
| $AUC_{0-last}$ (ng*hr/mL) | 2844±77.78* | 2039±81.9 |
| $AUC_{0-inf}$ (ng*hr/mL) | 2990±82.05* | 2186±82.1 |

The relative bioavailability was calculated by the following formula,

$$\text{The relative bioavailability (F)}=(AUC_T \times D_R)\div(AUC_R \times D_T)\times 100\%$$

wherein, AUC represents the area under the blood drug concentration-time curve ($AUC_{0\text{-inf}}$); D represents the administered dose; T and R represent Crystal Form A and Crystal Form B, respectively.

By calculation, it was found that the bioavailability of Crystal Form A relative to Crystal Form B was 137%, suggesting that the bioavailability of Crystal Form A is far superior over Crystal Form B.

The experimental results in Table 6 showed that the relevant pharmacokinetic parameters ($C_{max}$, $T_{max}$, $AUC_{0\text{-last}}$, $AUC_{0\text{-inf}}$) of Crystal Form A were significantly higher than those of Crystal Form B, with significant statistical differences ($P<0.05$), indicating that compared to Crystal Form B, Crystal Form A as pharmaceutical raw material can improve the bioavailability of the drug, prolong the action time of the drug, reduce the administration number and reduce the cost in clinical applications, and thus can be advantageous crystal form of pharmaceutical preparations.

## Claims

1. Crystal Form A of a compound of formula (1), wherein
the Crystal Form A has characteristic peaks at 4.46, 11.30, 13.59, 18.17, 21.38, 22.03, 25.89 in the X-ray powder diffraction pattern obtained by Cu-Kα radiation and represented by diffraction angle 2θ angle, wherein the error range of each characteristic peak 2θ is ±0.2,

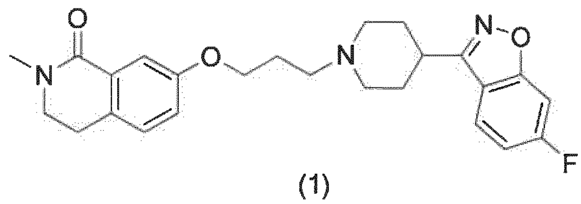

(1) .

2. The Crystal Form A according to claim 1, wherein
the Crystal Form A has an X-ray powder diffraction pattern having characteristic peaks, represented by 2θ angle, at 4.46, 9.01, 11.30, 12.55, 13.59, 14.21, 15.67, 16.45, 17.25, 18.17, 18.54, 18.85, 19.51, 20.89, 21.38, 22.03, 22.93, 24.43, 25.07, 25.89, 27.09, 27.81, 28.14, 29.31, 30.02 and 31.85, wherein the error range of each characteristic peak 2θ is ±0.2.

3. The Crystal Form A according to claim 2, wherein
the Crystal Form A has a Raman spectrum with characteristic peaks at 3065.5±2 $cm^{-1}$, 2958.4±2 $cm^{-1}$, 1607.8±2 $cm^{-1}$, 1447.8±2 $cm^{-1}$, 1320.2±2 $cm^{-1}$, 1271.5±2 $cm^{-1}$, 1125.3±2 $cm^{-1}$, 1009.3±2 $cm^{-1}$, 918.94±2 $cm^{-1}$, 714.8±2 $cm^{-1}$, 309.2±2 $cm^{-1}$, 233.2±2 $cm^{-1}$.

4. The Crystal Form A according to claim 3, wherein
the Crystal Form A has a DSC with melting endothermic peak values selected from the group consisting of 116.4 to 122.0°C, preferably 119.4°C.

5. A method for preparing the Crystal Form A according to any one of claims 1-4, comprising the following steps:

① dissolving a compound of formula (1) in a solvent to give a solution containing the compound of formula (1);
② removing the solvent in the solution obtained in step ① by evaporation method to give a precipitate;
wherein,
the solvent in step ① is selected from the group consisting of one or more of $C_{1-6}$ alcohol, ester, ketone, ether, halogenated hydrocarbon, N-methyl-2-pyrrolidone, $C_{5-10}$ saturated hydrocarbon, nitrile, water, N,N-dimethylformamide and dimethylsulfoxide;
the $C_{1-6}$ alcohol is selected from the group consisting of one or more of methanol, ethanol or propanol;
the ester is selected from the group consisting of one or two of ethyl acetate or methyl acetate;
the ketone is selected from the group consisting of one or two of acetone or butanone;
the ether is selected from the group consisting of one or two of methyl tert-butyl ether, ethyl ether or tetrahydrofuran;
the halogenated hydrocarbon is selected from the group consisting of one or more of dichloromethane or chloroform;
the $C_{5-10}$ saturated hydrocarbon is preferably one or two of n-hexane or n-heptane;
the nitrile is selected from the group consisting of acetonitrile.

**6.** A method for preparing the Crystal Form A according to any one of claims 1-4, comprising the following steps:

① dissolving a compound of formula (1) in a solvent to give a solution containing the compound of formula (1);
(2) obtaining a precipitate from the solution obtained in step ① by precipitation method;
wherein the solvent in step ① is selected from the group consisting of one or more of $C_{1-5}$ alcohol, ester, ketone, ether, halogenated hydrocarbon, N-methyl-2-pyrrolidone, $C_{5-10}$ saturated hydrocarbon, nitrile, water, N,N-dimethylformamide and dimethylsulfoxide;
the $C_{1-5}$ alcohol is selected from the group consisting of one or more of methanol, ethanol or propanol;
the ester is selected from the group consisting of one or two of ethyl acetate or methyl acetate;
the ketone is selected from the group consisting of one or two of acetone or butanone;
the ether is selected from the group consisting of one or two of methyl tert-butyl ether, ethyl ether or tetrahydrofuran;
the halogenated hydrocarbon is selected from the group consisting of one or more of dichloromethane or chloroform;
the $C_{5-10}$ saturated hydrocarbon is preferably one or two of n-hexane or n-heptane;
the nitrile is selected from the group consisting of acetonitrile;
the precipitation method is selected from the group consisting of cooling method or precipitant method;
the cooling method is subjecting the solution obtained in step ① to cooling process to precipitate the crystals out;
the precipitant method is adding a precipitant of the compound of formula (1) into the solution obtained in step ① to precipitate the crystals out.

**7.** The method for preparing the Crystal Form A according to claim 6, wherein

the cooling process is selected from the group consisting of lowering the temperature of the solution obtained in step ① to 0 to 60°C, preferably 10 to 40°C, more preferably 15 to 25°C; or
lowering the temperature to a temperature which is 20 to 100°C lower than that of the solution obtained in step ①, preferably 30 to 100°C lower than that of the solution obtained in step ①, more preferably 60 to 100°C lower than that of the solution obtained in step ①.

**8.** The method for preparing the Crystal Form A according to claim 6, wherein

the precipitant is selected from the group consisting of $C_{5-10}$ saturated alkane or water;
the $C_{5-10}$ saturated alkane is selected from the group consisting of one or more of n-pentane, n-hexane or n-heptane.

**9.** A pharmaceutical composition, comprising the Crystal Form A according to any one of claims 1-4, and one or more excipients, carriers, adjuvants, solvents or a combination thereof.

**10.** The Crystal Form A according to any one of claims 1-4 or the pharmaceutical composition according to claim 9 for use in treating and/or preventing a psychiatric disorder, preferably the psychiatric disorder is schizophrenia.

## Patentansprüche

**1.** Kristallform A einer Verbindung der Formel (1), wobei
die Kristallform A charakteristische Peaks bei 4,46, 11,30, 13,59, 18,17, 21,38, 22,03, 25,89 in dem Röntgenpulverbeugungsmuster aufweist, das durch Cu-K$\alpha$-Strahlung erhalten wurde und durch Beugungswinkel 2$\theta$-Winkel repräsentiert wird, wobei der Fehlerbereich jedes charakteristischen Peaks 2$\theta$ $\pm$ 0,2 beträgt,

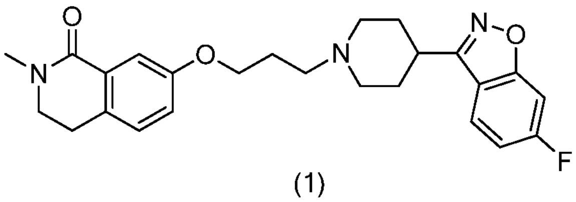

(1)

.

**2.** Kristallform A gemäß Anspruch 1, wobei die Kristallform A ein Röntgenpulverbeugungsmuster aufweist, das charakteristische Peaks aufweist, repräsentiert durch den 2θ-Winkel bei 4,46, 9,01, 11,30, 12,55, 13,59, 14,21, 15,67, 16,45, 17,25, 18,17, 18,54, 18,85, 19,51, 20,89, 21,38, 22,03, 22,93, 24,43, 25,07, 25,89, 27,09, 27,81, 28,14, 29,31, 30,02 und 31,85, wobei der Fehlerbereich jedes charakteristischen Peaks 2θ ± 0,2 beträgt.

**3.** Kristallform A gemäß Anspruch 2, wobei die Kristallform A ein Raman-Spektrum mit charakteristischen Peaks bei 3065,5 ± 2 $cm^{-1}$, 2958,4 ± 2 $cm^{-1}$, 1607,8 ± 2 $cm^{-1}$, 1447,8 ± 2 $cm^{-1}$, 1320,2 ± 2 $cm^{-1}$, 1271,5 ± 2 $cm^{-1}$, 1125,3 ± 2 $cm^{-1}$, 1009,3 ± 2 $cm^{-1}$, 918,94 ± 2 $cm^{-1}$, 714,8 ± 2 $cm^{-1}$, 309,2 ± 2 $cm^{-1}$, 233,2 ± 2 $cm^{-1}$ aufweist.

**4.** Kristallform A gemäß Anspruch 3, wobei die Kristallform A ein DSC mit Schmelzendothermie-Peakwerten, ausgewählt aus der Gruppe, bestehend aus 116,4 bis 122,0°C, vorzugsweise 119,4°C, aufweist.

**5.** Verfahren zum Herstellen der Kristallform A gemäß einem jeglichen der Ansprüche 1-4, umfassend die folgenden Schritte:

① Lösen einer Verbindung der Formel (1) in einem Lösungsmittel, um eine Lösung zu ergeben, die die Verbindung der Formel (1) enthält;
② Entfernen des Lösungsmittels in der in Schritt ① erhaltenen Lösung durch ein Verdampfungsverfahren, um ein Präzipitat zu ergeben,
wobei
das Lösungsmittel in Schritt ① ausgewählt ist aus der Gruppe, bestehend aus einem oder mehreren aus $C_{1-6}$-Alkohol, Ester, Keton, Ether, halogeniertem Kohlenwasserstoff, N-Methyl-2-pyrrolidon, gesättigtem $C_{5-10}$-Kohlenwasserstoff, Nitril, Wasser, N,N-Dimethylformamid und Dimethylsulfoxid;
der $C_{1-6}$-Alkohol ausgewählt ist aus der Gruppe, bestehend aus einem oder mehreren aus Methanol, Ethanol oder Propanol;
der Ester ausgewählt ist aus der Gruppe, bestehend aus einem oder zwei aus Ethylacetat oder Methylacetat;
das Keton ausgewählt ist aus der Gruppe, bestehend aus einem oder zwei aus Aceton oder Butanen;
der Ether ausgewählt ist aus der Gruppe, bestehend aus einem oder zwei aus Methyl-tert-butylether, Ethylether oder Tetrahydrofuran;
der halogenierte Kohlenwasserstoff ausgewählt ist aus der Gruppe, bestehend aus einem oder mehreren aus Dichlormethan oder Chloroform;
der gesättigte $C_{5-10}$-Kohlenwasserstoff ausgewählt ist aus der Gruppe, bestehend aus einem oder zwei aus n-Hexan oder n-Heptan;
das Nitril ausgewählt ist aus der Gruppe, bestehend aus Acetonitril.

**6.** Verfahren zum Herstellen der Kristallform A gemäß einem jeglichen der Ansprüche 1-4, umfassend die folgenden Schritte:

① Lösen einer Verbindung der Formel (1) in einem Lösungsmittel, um eine Lösung zu ergeben, die die Verbindung der Formel (1) enthält;
② Erhalten eines Präzipitats aus der in Schritt ① erhaltenen Lösung durch ein Präzipitationsverfahren;
wobei das Lösungsmittel in Schritt ① ausgewählt ist aus der Gruppe, bestehend aus einem oder mehreren aus $C_{1-5}$-Alkohol, Ester, Keton, Ether, halogeniertem Kohlenwasserstoff, N-Methyl-2-pyrrolidon, gesättigtem $C_{5-10}$-Kohlenwasserstoff, Nitril, Wasser, N,N-Dimethylformamid und Dimethylsulfoxid;
der $C_{1-5}$-Alkohol ausgewählt ist aus der Gruppe, bestehend aus einem oder mehreren aus Methanol, Ethanol oder Propanol;
der Ester ausgewählt ist aus der Gruppe, bestehend aus einem oder zwei aus Ethylacetat oder Methylacetat;

das Keton ausgewählt ist aus der Gruppe, bestehend aus einem oder zwei aus Aceton oder Butanen;
der Ether ausgewählt ist aus der Gruppe, bestehend aus einem oder zwei aus Methyl-tert-butylether, Ethylether oder Tetrahydrofuran;
der halogenierte Kohlenwasserstoff ausgewählt ist aus der Gruppe, bestehend aus einem oder mehreren aus Dichlormethan oder Chloroform;
der gesättigte $C_{5-10}$-Kohlenwasserstoff vorzugsweise eines oder zwei aus n-Hexan oder n-Heptan ist;
das Nitril ausgewählt ist aus der Gruppe, bestehend aus Acetonitril;
das Präzipitationsverfahren ausgewählt ist aus der Gruppe, bestehend aus einem Abkühlverfahren oder einem Fällungsmittelverfahren;
das Abkühlverfahren ein Aussetzen der in Schritt ① erhaltenen Lösung gegenüber einem Abkühlverfahren, um die Kristalle zu präzipitieren, ist;
das Fällungsmittelverfahren ein Zusetzen eines Fällungsmittels der Verbindung der Formel (1) in die in Schritt ① erhaltene Lösung, um die Kristalle zu präzipitieren, ist.

**7.** Verfahren zum Herstellen der Kristallform A gemäß Anspruch 6, wobei

das Abkühlverfahren ausgewählt ist aus der Gruppe, bestehend aus einem Absenken der Temperatur der in Schritt ① erhaltenen Lösung auf 0 bis 60°C, vorzugsweise 10 bis 40°C, mehr bevorzugt 15 bis 25°C; oder einem Absenken der Temperatur auf eine Temperatur ist, die 20 bis 100°C niedriger als die der in Schritt ① erhaltenen Lösung, vorzugsweise 30 bis 100°C niedriger als die der in Schritt ① erhaltenen Lösung, mehr bevorzugt 60 bis 100°C niedriger als die der in Schritt ① erhaltenen Lösung liegt.

**8.** Verfahren zum Herstellen der Kristallform A gemäß Anspruch 6, wobei

das Fällungsmittel ausgewählt ist aus der Gruppe, bestehend aus gesättigtem $C_{5-10}$-Alkan oder Wasser;
das gesättigte $C_{5-10}$-Alkan ausgewählt ist aus der Gruppe, bestehend aus einem oder mehreren aus n-Pentan, n-Hexan oder n-Heptan.

**9.** Pharmazeutische Zusammensetzung, die die Kristallform A gemäß einem jeglichen der Ansprüche 1-4 und ein(en) oder mehrere Hilfsstoffe, Träger, Adjuvanzien, Lösungsmittel oder eine Kombination davon umfasst.

**10.** Kristallform A gemäß einem jeglichen der Ansprüche 1-4 oder pharmazeutische Zusammensetzung gemäß Anspruch 9 zur Verwendung beim Behandeln und/oder Verhindern einer psychiatrischen Störung, wobei vorzugsweise die psychiatrische Störung Schizophrenie ist.

## Revendications

**1.** Forme cristalline A d'un composé de formule (1), dans laquelle
la forme cristalline A présente des pics caractéristiques à 4,46, 11,30, 13,59, 18,17, 21,38, 22,03, 25,89 dans le diagramme de diffraction des rayons X sur poudre obtenu par rayonnement Cu-K$\alpha$ et représenté par l'angle de diffraction $2\theta$, dans laquelle l'intervalle d'erreur de chaque pic caractéristique $2\theta$ est de $\pm0,2$,

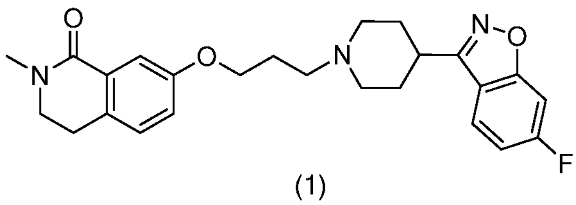

(1)

**2.** Forme cristalline A selon la revendication 1, dans laquelle
la forme cristalline A présente un diagramme de diffraction des rayons X sur poudre avec des pics caractéristiques, représentés par l'angle $2\theta$, à 4,46, 9,01, 11,30, 12,55, 13,59, 14,21, 15,67, 16,45, 17,25, 18,17, 18,54, 18,85, 19,51, 20,89, 21,38, 22,03, 22,93, 24,43, 25,07, 25,89, 27,09, 27,81, 28,14, 29,31, 30,02 et 31,85, l'intervalle d'erreur de chaque pic caractéristique $2\theta$ étant de $\pm0,2$.

**3.** Forme cristalline A selon la revendication 2, dans laquelle

la forme cristalline A présente un spectre Raman avec des pics caractéristiques à 3065,5±2 $cm^{-1}$, 2958,4±2 $cm^{-1}$, 1607,8±2 $cm^{-1}$, 1447,8±2 $cm^{-1}$, 1320,2±2 $cm^{-1}$, 1271,5±2 $cm^{-1}$, 1125,3±2 $cm^{-1}$, 1009,3±2 $cm^{-1}$, 918,94±2 $cm^{-1}$, 714,8±2 $cm^{-1}$, 309,2±2 $cm^{-1}$, 233,2±2 $cm^{-1}$.

**4.** Forme cristalline A selon la revendication 3, dans laquelle
la forme cristalline A présente une DSC avec des valeurs de pic endothermique de fusion choisies dans le groupe constitué de 116,4 à 122,0 °C, de préférence 119,4 °C.

**5.** Procédé de préparation de la forme cristalline A selon l'une quelconque des revendications 1 à 4, comprenant les étapes suivantes :

① dissolution d'un composé de formule (1) dans un solvant pour donner une solution contenant le composé de formule (1) ;
② élimination du solvant de la solution obtenue à l'étape ① par évaporation pour obtenir un précipité ;
dans lequel,
le solvant à l'étape ① est choisi dans le groupe constitué d'un ou de plusieurs éléments parmi un alcool en $C_{1-6}$, un ester, une cétone, un éther, un hydrocarbure halogéné, de la N-méthyl-2-pyrrolidone, un hydrocarbure saturé en $C_{5-10}$, du nitrile, de l'eau, du N,N-diméthylformamide et du diméthylsulfoxyde ;
l'alcool en $C_{1-6}$ est choisi dans le groupe constitué d'un ou de plusieurs éléments parmi le méthanol, l'éthanol ou le propanol ;
l'ester est choisi dans le groupe constitué d'un ou deux éléments parmi l'acétate d'éthyle ou l'acétate de méthyle ;
la cétone est choisie dans le groupe constitué d'un ou deux éléments parmi l'acétone ou la butanone ;
l'éther est choisi dans le groupe constitué d'un ou deux éléments parmi le tert-butyl-éther de méthyle, l'éther éthylique ou le tétrahydrofurane ;
l'hydrocarbure halogéné est choisi dans le groupe constitué d'un ou de plusieurs éléments parmi un dichlorométhane ou un chloroforme ;
l'hydrocarbure saturé en $C_{5-10}$ est de préférence un ou deux éléments parmi le n-hexane ou le n-heptane ;
le nitrile est sélectionné dans le groupe constitué d'acétonitrile.

**6.** Procédé de préparation de la forme cristalline A selon l'une quelconque des revendications 1 à 4, comprenant les étapes suivantes :

① dissolution d'un composé de formule (1) dans un solvant pour donner une solution contenant le composé de formule (1) ;
② l'obtention d'un précipité à partir de la solution obtenue à l'étape ① par la méthode de précipitation ;
dans lequel le solvant à l'étape ① est choisi dans le groupe constitué d'un ou de plusieurs éléments parmi un alcool en $C_{1-5}$, un ester, une cétone, un éther, un hydrocarbure halogéné, de la N-méthyl-2-pyrrolidone, un hydrocarbure saturé en $C_{5-10}$, du nitrile, de l'eau, du N,N-diméthylformamide et du diméthylsulfoxyde ;
l'alcool en $C_{1-5}$ est choisi dans le groupe constitué d'un ou de plusieurs éléments parmi le méthanol, l'éthanol ou le propanol ;
l'ester est choisi dans le groupe constitué d'un ou deux éléments parmi l'acétate d'éthyle ou l'acétate de méthyle ;
la cétone est choisie dans le groupe constitué d'un ou deux éléments parmi l'acétone ou la butanone ;
l'éther est choisi dans le groupe constitué d'un ou deux éléments parmi le tert-butyl-éther de méthyle, l'éther éthylique ou le tétrahydrofurane ;
l'hydrocarbure halogéné est choisi dans le groupe constitué d'un ou de plusieurs éléments parmi un dichlorométhane ou un chloroforme ;
l'hydrocarbure saturé en $C_{5-10}$ est de préférence un ou deux éléments parmi le n-hexane ou le n-heptane ;
le nitrile est sélectionné dans le groupe constitué d'acétonitrile ;
le procédé de précipitation est choisi dans le groupe constitué par la méthode de refroidissement ou la méthode de précipitation ;
le procédé de refroidissement consiste à soumettre la solution obtenue à l'étape ① à un processus de refroidissement afin de précipiter les cristaux ;
le procédé du précipité consiste à ajouter un précipité du composé de formule (1) dans la solution obtenue à l'étape ① afin de précipiter les cristaux.

**7.** Procédé de préparation de la forme cristalline A selon la revendication 6,
dans lequel

le processus de refroidissement est choisi dans le groupe consistant à abaisser la température de la solution obtenue à l'étape ① entre 0 et 60 °C, de préférence entre 10 et 40 °C, plus préférentiellement entre 15 et 25 °C ; ou abaisser la température à une température inférieure de 20 à 100 °C à celle de la solution obtenue à l'étape ①, de préférence de 30 à 100 °C inférieure à celle de la solution obtenue à l'étape ①, de manière davantage préférée, de 60 à 100 °C inférieure à celle de la solution obtenue à l'étape ①.

**8.** Procédé de préparation de la forme cristalline A selon la revendication 6, dans lequel

le précipité est choisi dans le groupe constitué d'alcane saturé en $C_{5-10}$ ou d'eau ;
l'alcane saturé en $C_{5-10}$ est choisi dans le groupe constitué d'un ou de plusieurs éléments parmi le n-pentane, le n-hexane ou le n-heptane.

**9.** Composition pharmaceutique comprenant la forme cristalline A selon l'une quelconque des revendications 1 à 4, et un ou plusieurs excipients, supports, adjuvants, solvants ou une combinaison de ceux-ci.

**10.** Forme cristalline A selon l'une quelconque des revendications 1 à 4 ou composition pharmaceutique selon la revendication 9 pour une utilisation dans le traitement et/ou la prévention d'un trouble psychiatrique, de préférence la schizophrénie.

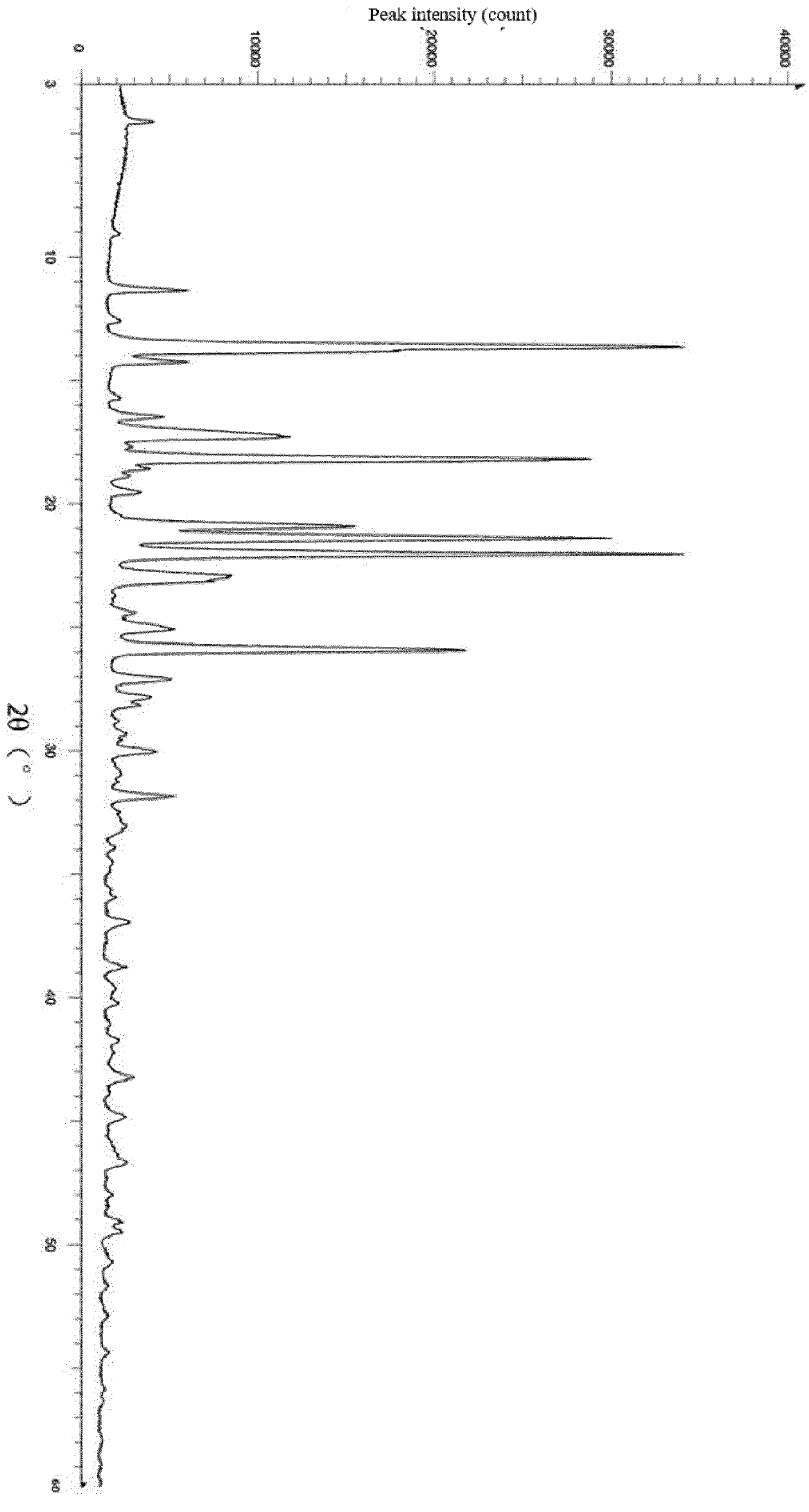
Peak intensity (count)
0
10000
20000
30000
40000
2θ (°)
3
10
20
30
40
50
60

Figure 1

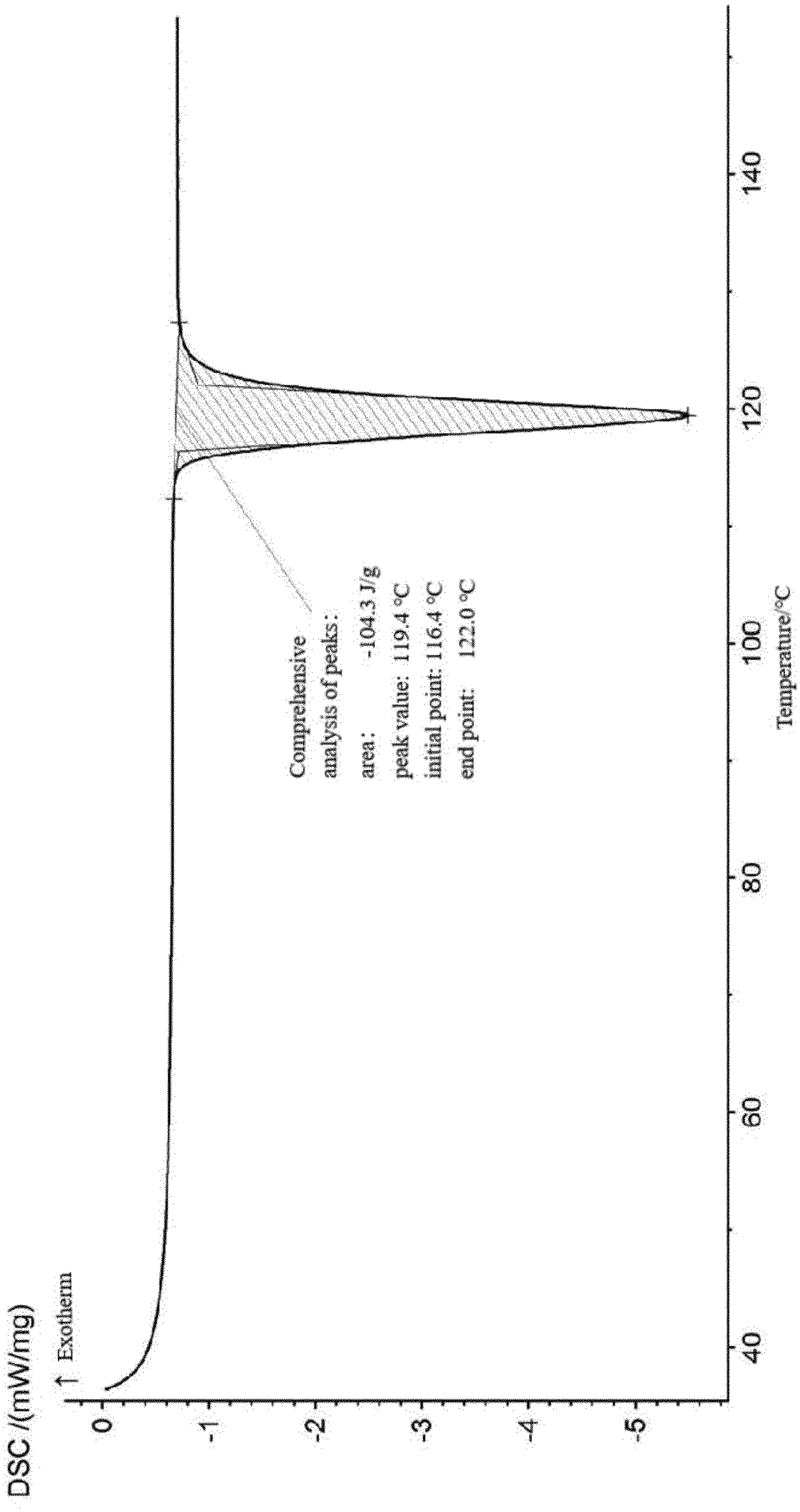
DSC /(mW/mg)
↑ Exotherm
0
-1
-2
-3
-4
-5
40
60
80
100
120
140
Temperature/°C
Comprehensive
analysis of peaks:
area: -104.3 J/g
peak value: 119.4 °C
initial point: 116.4 °C
end point: 122.0 °C

Figure 2

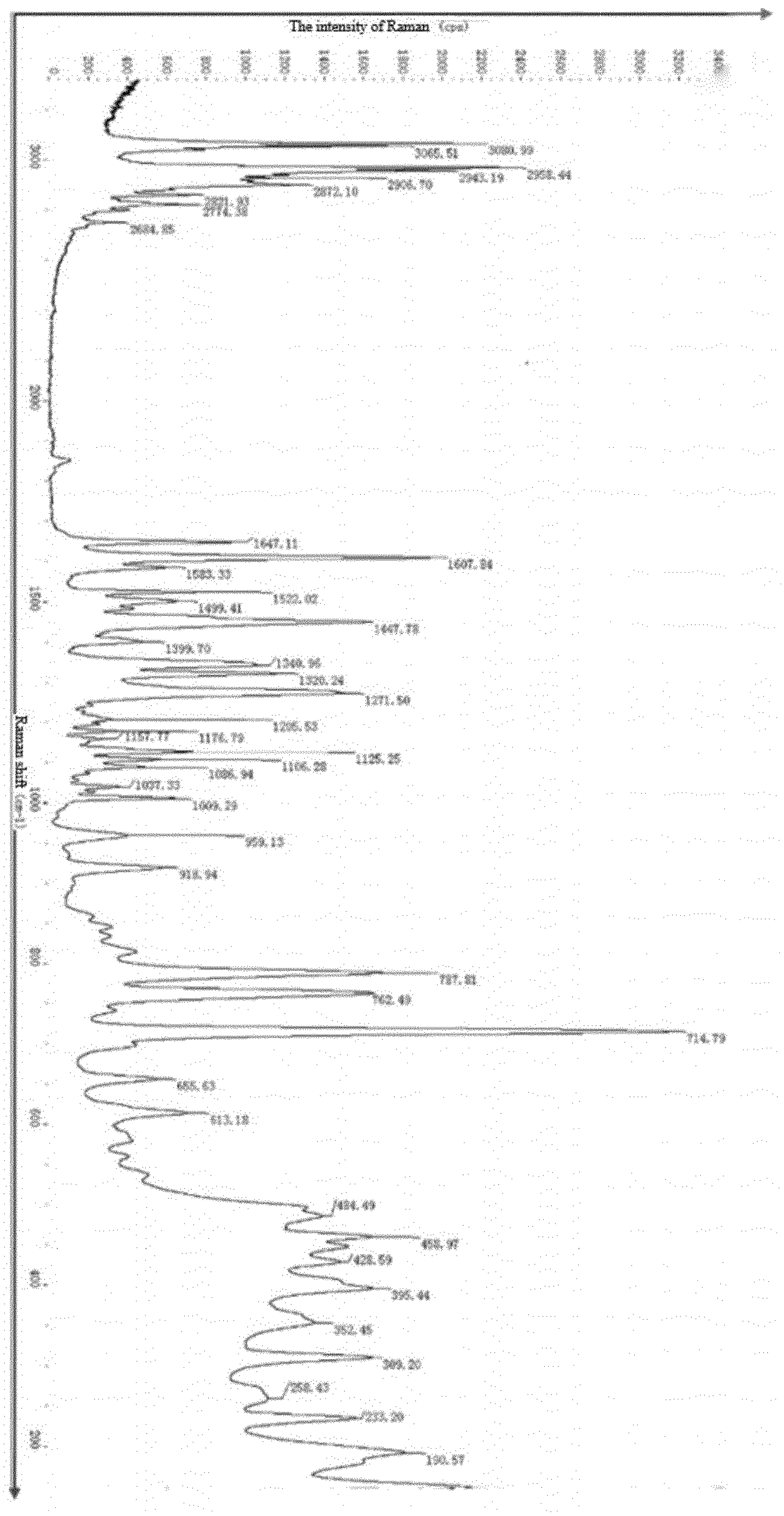
The intensity of Raman (cps)
Raman shift (cm-1)
3065.51
3080.99
2958.44
2943.19
2905.70
2872.10
2774.58
2684.88
1647.11
1607.84
1583.33
1522.02
1499.41
1447.78
1399.70
1340.96
1320.24
1271.50
1205.53
1157.77
1176.79
1125.25
1106.28
1086.94
1037.33
1009.29
959.13
918.94
787.81
762.49
714.79
655.63
613.18
484.49
458.97
428.59
395.44
352.45
309.20
258.43
233.20
190.57

Figure 3

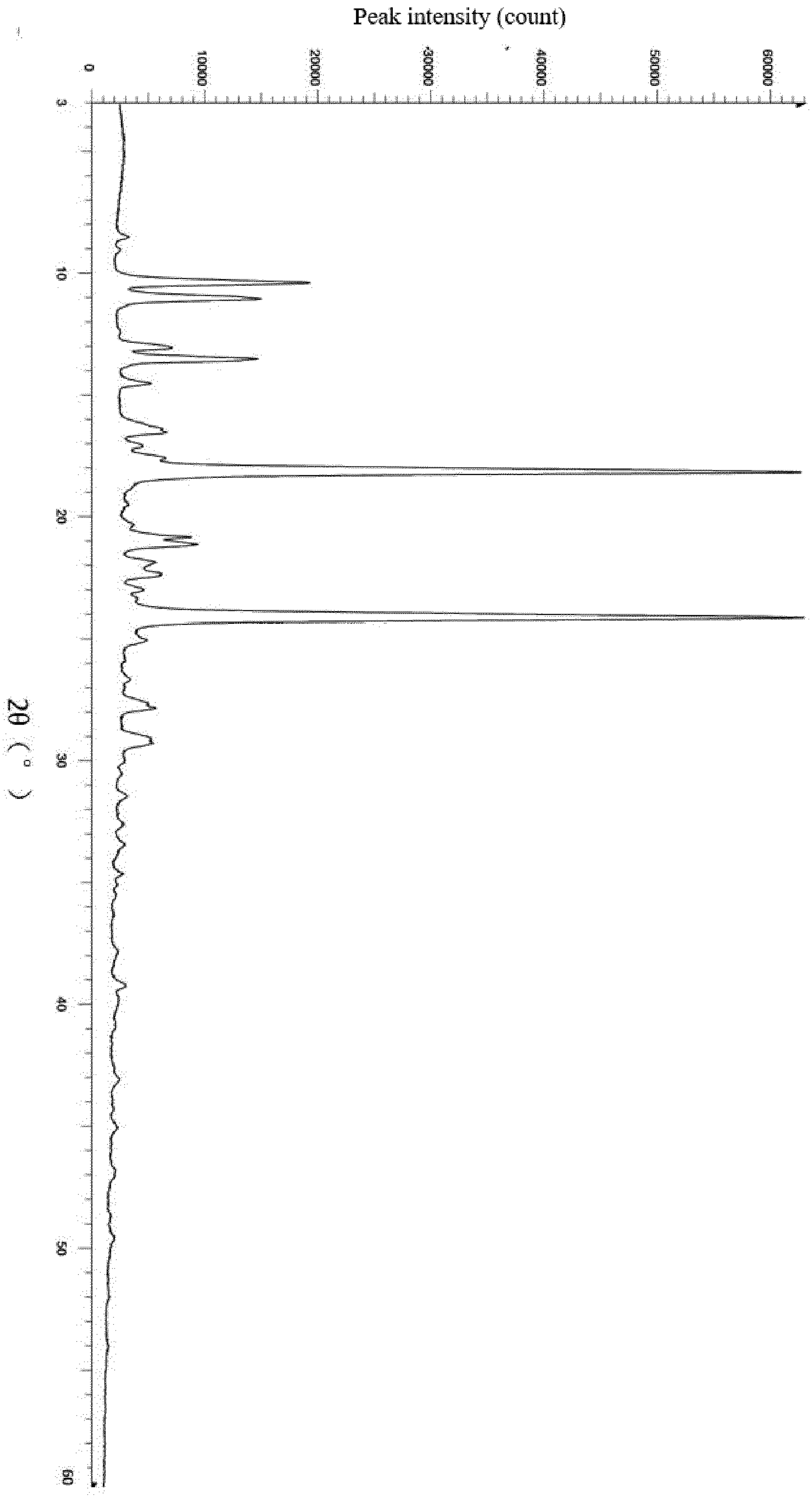
Peak intensity (count)
0
10000
20000
30000
40000
50000
60000
3
10
20
30
40
50
60
2θ (°)

Figure 4

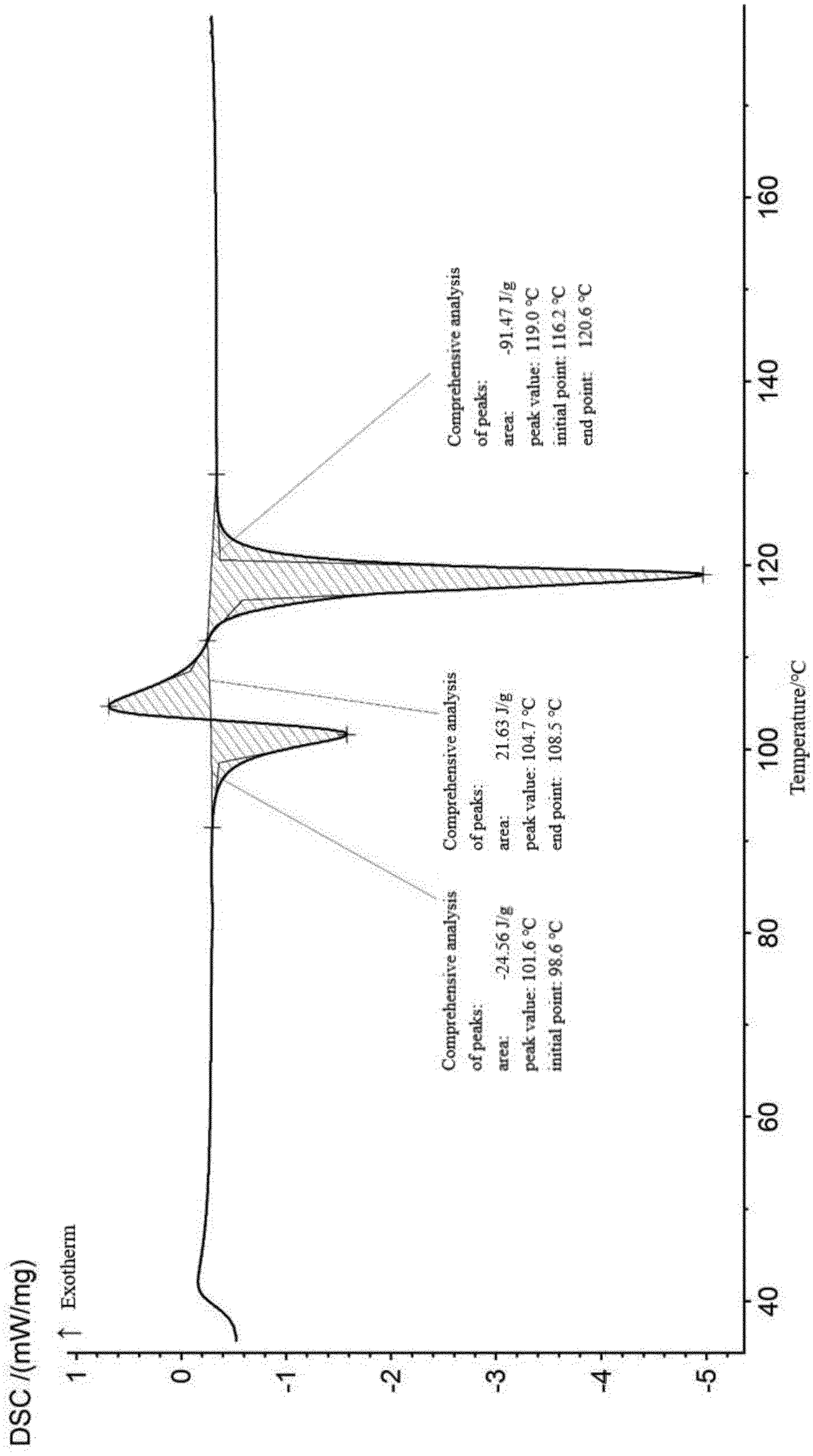
DSC /(mW/mg)
↑ Exotherm
Temperature/°C
Comprehensive analysis of peaks:
area: -24.56 J/g
peak value: 101.6 °C
initial point: 98.6 °C
Comprehensive analysis of peaks:
area: 21.63 J/g
peak value: 104.7 °C
end point: 108.5 °C
Comprehensive analysis of peaks:
area: -91.47 J/g
peak value: 119.0 °C
initial point: 116.2 °C
end point: 120.6 °C

Figure 5

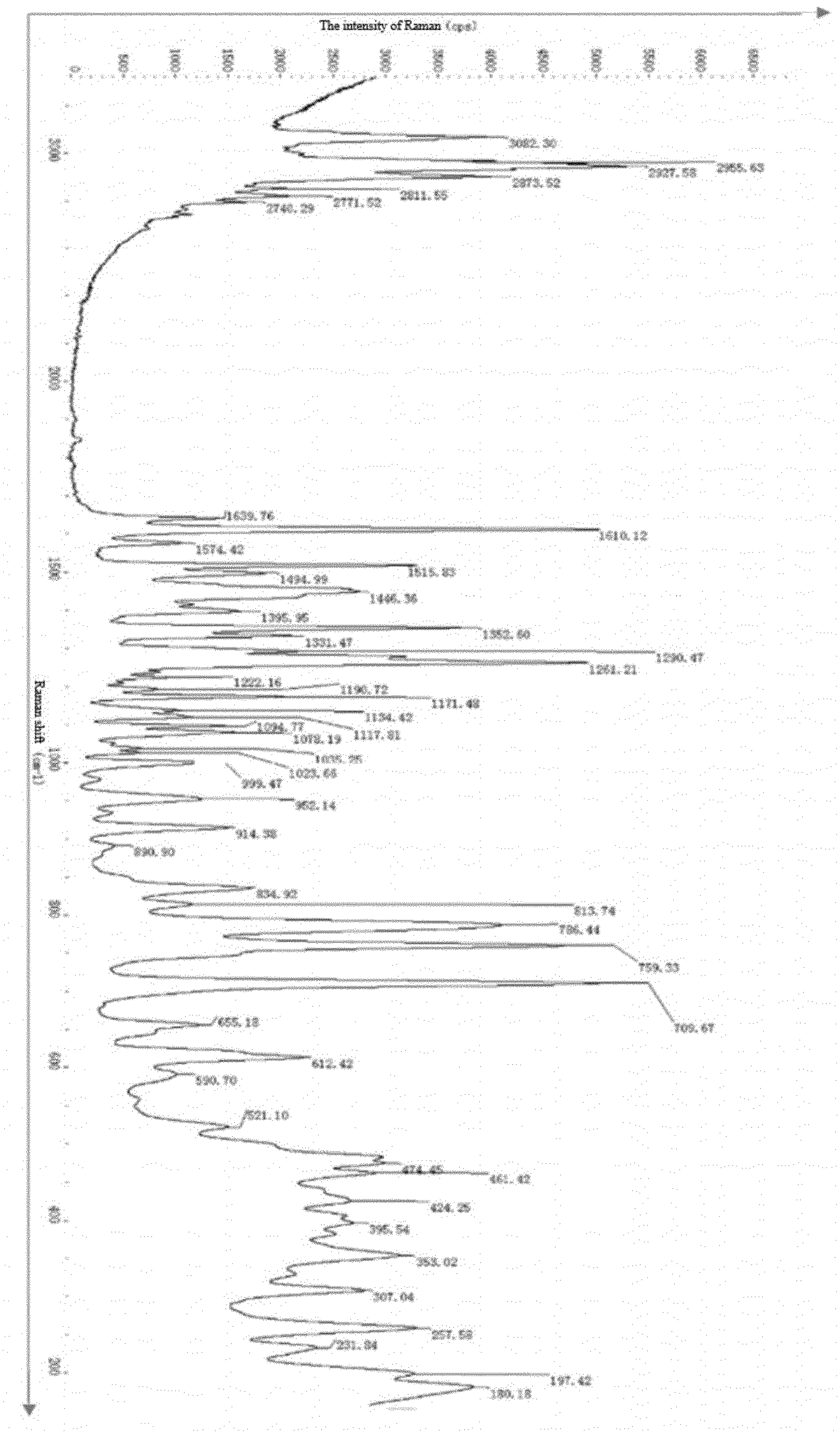
The intensity of Raman (cps)
Raman shift (cm-1)
3082.30
2927.58
2955.63
2873.52
2811.55
2771.52
2740.29
1639.76
1610.12
1574.42
1515.83
1494.99
1446.36
1395.95
1352.60
1331.47
1290.47
1261.21
1222.16
1190.72
1171.48
1134.42
1094.77
1117.81
1078.19
1035.25
1023.66
999.47
952.14
914.38
890.90
834.92
813.74
786.44
759.33
709.67
655.18
612.42
590.70
521.10
474.45
461.42
424.25
395.54
353.02
307.04
257.58
231.84
197.42
180.18

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017084627 A1
- CN 2016106591 W


### Non-patent literature cited in the description


- Chinese Pharmacopoeia. 2015
- **GABRIELSSON J ; WEINER D.** Pharmacokinetic and pharmacodynamic data analysis: concepts and applications[M. CRC Press, 2001, 141-146